# EUROPEAN PATENT APPLICATION

(11) **EP 4 295 872 A1**
(43) Date of publication of application: **27.12.2023**
(21) Application number: 23178504.9
(22) Date of filing: 09.06.2023
(51) Int. Cl.: A61M 1/34, A61M 1/36

(54) **BLOOD TREATMENT APPARATUS**

(30) Priority: 22.06.2022 IT 202200013168; 08.02.2023 IT 202300002148
(71) Applicant: Purotech S.r.l., 40125 Bologna (IT)
(72) Inventor: CALLETTI, Filippo, BOLOGNA (IT)
(74) Representative: Puggioli, Tommaso

(57) **Abstract**

An apparatus (1) for blood treatment comprising an extracorporeal circuit (2) comprising: a filter (3) designed to separate the blood of a patient (P) into a plasma component and into a cardiovascular component, an arterial line (4), positioned upstream of the filter (3), for conveying the blood from the patient (P) to the filter (3), a treatment device (6) positioned downstream of the filter (3) and wherein said plasma component is treated with a gas comprising ozone and/or oxygen to obtain a treated plasma component, an infeed line (7) positioned between the filter (3) and the treatment device (6) for conveying the plasma component from the filter (3) to the treatment device (6), a mixing device (16) located downstream of the filter (3), according to a feed direction of the corpuscular component, and of the treatment device (6) according to the feed direction of the plasma component, an outfeed line (8) downstream of the treatment device (6) for transferring the treated plasma component from the treatment device (6) to the mixing device (16), said mixing device (16) being in communication with the treatment device (6) by means of the outfeed line (8) and with the filter (3) for receiving the treated plasma component and the corpuscular component, said mixing device (16) being configured for mixing the corpuscular component and the treated plasma component and providing as output a treated blood, the extracorporeal circuit (2) comprising a venous line (5) located downstream of the mixing device (16) for conveying the treated blood to the patient (P), said treatment device (6) comprising an ozone generator (12) passed through by said plasma component, configured to generate in line in the extracorporeal circuit (2), ozone in said plasma component.

## Description

This invention relates to an apparatus for the blood treatment of a patient. In particular, the invention relates to the sector of blood treatment with gas, for example with oxygen and/or ozone.

Extracorporeal treatment systems are known in the prior art with ozone and/or oxygen in gaseous form.

For example, a first type of blood treatment system belongs to the self-blood transfusion category in which approximately 200-250 ml of blood are taken by the patient and transferred inside a transfusion bag; the blood inside the bag is then oxygenated-ozonated and then reinfused. However, this first type of system has several drawbacks. In particular, the limited quantity of blood treated for a treatment session does not allow an optimum effect to be obtained in the body, and it is therefore necessary to perform several treatment sessions, with inevitable negative consequences from the point of view of the patient.

A second type of blood treatment system involves putting the blood in contact with oxygen and ozone which is specially produced. Disadvantageously, this technique has problems in dissolving these gases inside the blood, considerably adversely affecting the blood treatment. The aim of the invention is to provide an apparatus for blood treatment which overcomes the above-mentioned disadvantages of the prior art. Said aim is achieved by the apparatus for blood treatment according to the invention as characterised in the appended claims.

In particular, according to the invention, an apparatus is made for blood treatment, as defined in claim 1 or as defined in claim 8.

The apparatus is applicable to the extracorporeal treatment of the blood of a person by means of a gas comprising gaseous ozone and/or gaseous oxygen.

The apparatus for the treatment of blood makes it possible to continuously collect a flow of blood, separate the plasma component from the corpuscular component, treat the plasma component with ozone and/or oxygen (preferably generated in line), mix the treated plasma component and the corpuscular component which generates a treated flow of blood, re-infusing the flow of treated blood in the circulating system of the patient. Said treatment of the blood by means of ozone and/or oxygen presents a plurality of beneficial biological effects, including: bactericidal and virucidal action against pathogens present in the blood; analgesic and antiinflammatory action; strengthening and regulation of the immune system; improvement of blood circulation (optimising the functions of organs and tissues).

These biological effects can be obtained in very small and extremely controlled concentrations of ozone and/or oxygen (as described in more detail below); these reduced concentrations are necessary due to the high toxicity of ozone and/or oxygen for the treated person (when ozone and/or oxygen comes into contact with the blood or with bodily tissues). According to an aspect of the invention, the invention relates to an apparatus for blood treatment which comprises an extracorporeal circuit. The extracorporeal circuit comprises:
- a filter designed to separate the blood of a patient into a plasma component and into a corpuscular component,
- an arterial line, located upstream of the filter, in a direction of feeding the blood of the patient, for conveying the blood from the patient to the filter.
   - a treatment device located downstream of the filter, in a direction of feeding the plasma component, and wherein the plasma component is treated with a gas comprising ozone and/or oxygen to obtain a treated plasma component,
   - an infeed line located between the filter and the treatment device for conveying the plasma component from the filter to the treatment device,
   - a mixing device located downstream of the filter in a direction of feeding the corpuscular component, and of the treatment device in the direction of feeding the plasma component,
   - an outfeed line downstream of the treatment device, in the direction of feeding the plasma component, for transferring the treated plasma component from the treatment device to the mixing device.

The mixing device is in communication with the treatment device by means of the outfeed line and with the filter for receiving, respectively, the treated plasma component and the corpuscular component The mixing device is configured to mix the corpuscular component and the treated plasma component and provide at the outlet a treated blood.

The extracorporeal circuit comprises a venous line located downstream of the mixing device, in a direction of feeding the treated blood, for conveying the treated blood to the patient.

The treatment device comprises an ozone generator passed through by the plasma component, configured for generating in line in the extracorporeal circuit ozone and/or oxygen and dissolving ozone and/or oxygen in said plasma component.

The expression "in line" means that the gas, that is, the ozone and/or the oxygen, is generated in situ inside the extracorporeal circuit.

In fact, it should be noted that the extracorporeal circuit is a closed circuit; the blood is extracted from the patient and the plasma component is treated inside the circuit itself and the blood treated is re-infused to the patient.

According to an aspect, the ozone generator is an electrolysis ozone generator.

Advantageously, the ozone generator operating by electrolysis makes it possible to eliminate the dependency on a source of air and oxygen for producing ozone, allowing the production of ozone directly in the plasma component.

According to an aspect, the ozone generator comprises a body having a main direction of extension, an inlet for the plasma component and an outlet for the treated plasma component.

The ozone generator also comprises:
- at least one cathode inserted inside the main body and having a respective positive power supply terminal,
- at least one anode inserted in the main body and having a respective negative power supply terminal,
- an ion exchange membrane positioned between the cathode and the anode,
- a power supply unit for said cathode and anode.

The cathode, the anode and the exchange membrane intercept the plasma component passing through the main body.

The membrane extends mainly along the main direction of extension in such a way as to be interposed between the first and the second plate. According to an aspect, the treatment device comprises a computerised command and control unit.

The computerised command and control unit is in communication with at least the power supply unit to regulate the power supply voltage.

By adjusting the power supply voltage it is possible to act on the quantity of ozone and/or oxygen produced in the plasma component.

The cathode may comprise a first plate extending along the main direction of extension.

Preferably, the first plate has a plurality of through holes through a thickness of the first plate.

The anode may comprise a second plate extending along the main direction of extension.

According to an example, each through hole of the plurality of through holes is V-shaped.

Advantageously, the "V" shape of the holes allows an efficient action for breaking the gas bubbles comprising oxygen in the plasma component, favouring the formation of hydrogen inside it.

According to an aspect, the treatment device may comprise a flow meter positioned upstream of the ozone generator in the feed direction of the plasma component.

The flow meter is configured to measure a flow of plasma component entering the treatment device and generate a first signal representing the flow measured.

In other words, the flow meter makes it possible to measure the flow rate of the plasma component to be treated.

The flow meter is in communication with the computerised command and control unit.

The computerised command and control unit is configured to control the power supply unit as a function of the first signal.

According to an aspect, the treatment device may comprise an ozone flow meter positioned downstream of the ozone generator in the feed direction of the plasma component.

The ozone flow meter is configured for measuring a concentration of ozone in the treated plasma component and to generate a second signal signifying the measured ozone concentration.

The ozone flow meter is in communication with the computerised command and control unit.

The computerised command and control unit is configured to control the power supply unit as a function of the second signal.

Advantageously, the ozone measuring device makes it possible to monitor that the ozone concentration in the treated plasma component is within the range of 0.5 ppm - 50 ppm.

According to an aspect, the treatment device may comprise a thermometer configured to measure a temperature of the treated plasma component and in communication with the computerised command and control unit. Preferably, the thermometer is located downstream of the ozone generator.

The thermometer is configured to generate a third signal signifying the measured temperature and the computerised command and control unit is configured to control the power supply unit as a function of the third signal. According to an aspect, the extracorporeal circuit may comprise a device for adjusting the temperature positioned downstream of the treatment device, according to the feed direction of the plasma component.

The temperature adjustment device is in communication with the computerised command and control unit of the treatment device.

The computerised command and control unit is configured to control the temperature adjustment device as a function of the third signal.

According to an aspect, the extracorporeal circuit comprises in the infeed line to the treatment device a first pump configured to move the plasma component towards the treatment device.

Advantageously, the adjustment device allows a treated blood to be re-infused to the patient at a temperature in the range of 36°C - 38°C. Preferably, the first pump is in communication with the computerised unit which is configured to control the first pump.

According to an aspect, the extracorporeal circuit comprises in the arterial line a second pump configured to move the blood towards the filter. Preferably, the second pump is in communication with the computerised unit which is configured to control the second pump.

The extracorporeal circuit preferably comprises a trap device located downstream of the mixing device, in a direction of feeding the treated blood.

The trap device is configured for detecting and trapping bubbles of gas present inside the treated blood.

According to an aspect of the invention, the invention relates to an apparatus for blood treatment which comprises an extracorporeal circuit. The extracorporeal circuit comprises:
- a filter designed to separate the blood of a patient into a plasma component and into a corpuscular component,
- an arterial line, located upstream of the filter, in a direction of feeding the blood of the patient, for conveying the blood from the patient to the filter,
- a treatment unit located downstream of the filter, in a feed direction of the plasma component, the treatment unit comprising a treatment duct with an inlet section and an outlet section.

The treatment duct comprises an ozonizing stretch in which the plasma component is treated to obtain a treated plasma component.

The treatment unit comprises first and second coupling means at the inlet section and the outlet section, respectively.

The treatment duct is coupled to the filter by the first coupling means.

The extracorporeal circuit comprises a mixing device in communication with the outlet of the treatment duct and with the filter.

The mixing device is configured to mix the corpuscular component and the treated plasma component and provide at the outlet a treated blood.

The treatment duct is coupled to the mixing device by the second coupling means.

The extracorporeal circuit comprises:
- a venous line located downstream of the mixing device, in a direction of feeding the treated blood, for conveying the treated blood to the patient,
- a UV ray emitter associated with the treatment unit in such a way as to emit a radiation in the direction of the ozonizing stretch and to generate in line in the ozonizing stretch, ozone and/or oxygen in the plasma component in transit along the treatment duct.

The expression "in line" means that the gas, that is, the ozone and/or the oxygen, is generated in situ inside the extracorporeal circuit.

In fact, it should be noted that the extracorporeal circuit is a closed circuit; the blood is extracted from the patient and the plasma component is treated inside the circuit itself and the blood treated is re-infused to the patient.

The extracorporeal circuit comprises a computerised command and control unit in communication with the UV ray emitter to adjust an intensity of the radiation emitted.

Advantageously, by adjusting the intensity of the radiation emitted it is possible to act on the quantity of ozone and/or oxygen produced in the plasma component.

According to an aspect, the UV ray emitter is a UV-C ray emitter.

According to an aspect, the UV ray emitter comprises a quartz lamp.

According to an aspect, the UV ray emitter comprises an LED lamp.

According to an example, the UV ray emitter may face the ozonizing stretch.

According to an example, the UV ray emitter may be positioned in such a way as to completely surround the ozonizing stretch.

According to an example, the UV ray emitter may be positioned in such a way as to partly surround the ozonizing stretch.

According to an aspect, the ozonizing stretch is made of glass, for example quartz glass, or of plastic material, such as, for example, PVC. The material with which the piece is made must be complementary/compatible with UV-C radiation.

According to an aspect, the treatment unit may comprise a flow meter positioned upstream of the ozone generator in the feed direction of the plasma component.

The flow meter is configured to measure a flow of plasma component in transit in the treatment duct and generate a first signal representing the flow measured.

In other words, the flow meter makes it possible to measure the flow rate of the plasma component to be treated.

The flow meter is in communication with the computerised command and control unit.

The computerised command and control unit is configured to adjust the intensity of the radiation emitted as a function of the first signal.

According to an aspect, the treatment unit may comprise an ozone flow meter positioned downstream of the UV ray emitter in the feed direction of the plasma component.

The ozone flow meter is configured for measuring a concentration of ozone in the treated plasma component and to generate a second signal signifying the measured ozone concentration.

The ozone flow meter is in communication with the computerised command and control unit.

The computerised command and control unit is configured to adjust the intensity of UV rays of the radiation emitted as a function of the second signal.

Advantageously, the ozone measuring device makes it possible to monitor that the ozone concentration in the treated plasma component is within the range of 0.5 ppm - 50 ppm.

According to an aspect, the treatment unit may comprise a thermometer configured to measure a temperature of the treated plasma component. The thermometer is in communication with the computerised command and control unit.

Preferably, the thermometer is located downstream of the UV ray emitter. The thermometer is configured to generate a third signal signifying the measured temperature.

According to an aspect, the extracorporeal circuit may comprise a device for adjusting the temperature positioned downstream of the treatment unit, according to the feed direction of the plasma component.

The temperature adjustment device is in communication with the computerised control unit.

Advantageously, the adjustment device allows a treated blood to be re-infused to the patient at a temperature in the range of 36°C - 38°C. According to an aspect, the treatment unit comprises a first pump configured to move the plasma component in the treatment duct. Preferably, the first pump is in communication with the computerised unit which is configured to control the first pump.

According to an aspect, the extracorporeal circuit comprises in the arterial line a second pump configured to move the blood towards the filter. Preferably, the second pump is in communication with the computerised unit which is configured to control the second pump.

The extracorporeal circuit preferably comprises a trap device located downstream of the mixing device, in a direction of feeding the treated blood.

The trap device is configured for detecting and trapping bubbles of gas present inside the treated blood.

According to an aspect, the treatment duct comprises:
- a flow meter, positioned upstream of the UV ray emitter according to the feed direction of the plasma component, in communication with the computerised command and control unit, configured for measuring a flow of plasma component in the treatment duct and generating a first signal representing the measured flow;
- a device for measuring ozone in said treated plasma component, positioned downstream of the UV ray emitter according to the feed direction of the plasma component, in communication with said computerised command and control unit, the ozone measuring device is configured for measuring a concentration of ozone in the treated plasma component and generating a second signal representing the measured ozone concentration, whilst computerised command and control unit is configured for adjusting the intensity of UV rays of the radiation emitted as a function of said second signal;
- a thermometer configured to measure a temperature of the treated plasma component, in communication with said computerised command and control unit, configured to generate a third signal representing the measured temperature,
- a first pump configured to move the plasma component in the treatment duct in communication with the command and control unit is configured to control the first pump as a function of said first signal.

According to an aspect, the treatment unit is separable from the extracorporeal circuit.

The treatment unit can therefore be replaced in the extracorporeal circuit. Advantageously, the treatment unit may be replaced for each patient, thus guaranteeing a greater level of hygiene.

Moreover, having a treatment unit which can be replaced facilitates the cleaning and maintenance of the machine.

According to an aspect of the invention, the invention relates to a single-use treatment unit for a blood treatment apparatus as described above. The single-use treatment unit comprises the treatment duct comprising the ozonizing section in which the plasma component is treated to obtain a treated plasma component.

The treatment unit comprises first and second coupling means at the inlet section and the outlet section of the treatment duct, respectively.

The treatment duct can be coupled to the filter and to the mixing device of the blood treatment apparatus using the first and second coupling means. The ozonizing stretch is made of a material compatible with UV radiation, in particular with UV-C radiation

Advantageously, making a single-use treatment unit increases the level of safety and hygiene for the patient.

For example, the ozonising stretch is made of glass or plastic material. According to an aspect, the treatment duct comprises:
- a flow meter configured for measuring a flow of plasma component in said treatment duct and generating a first signal representing the flow measured;
- and/or ozone measuring device configured for measuring a concentration of ozone in the treated plasma component and generating a second signal representing the measured ozone concentration;
- and/or a thermometer configured to measure a temperature of the treated plasma component and generate a third signal representing the measured temperature;
- and/or a pump configured to move the plasma component in the treatment duct.

The features of the invention are more apparent from the detailed description which follows, with reference to the accompanying drawings which illustrate a preferred embodiment of the invention purely by way of non-limiting example, and in which:
- Figure 1A is a schematic view of a first embodiment of an apparatus for blood treatment according to the invention;
- Figure 1B is a schematic view of a treatment device forming part of the apparatus of Figure 1A;
- Figure 1C is a schematic view of an ozone generator forming part of said treatment device of Figure 1B.
- Figure 2A is a schematic view of a second embodiment of an apparatus for blood treatment according to the invention;
- Figure 2B illustrates a detail of the blood treatment apparatus of Figure 2A;
- Figure 2C is a schematic view of a single-use treatment unit for the apparatus of Figure 2A.

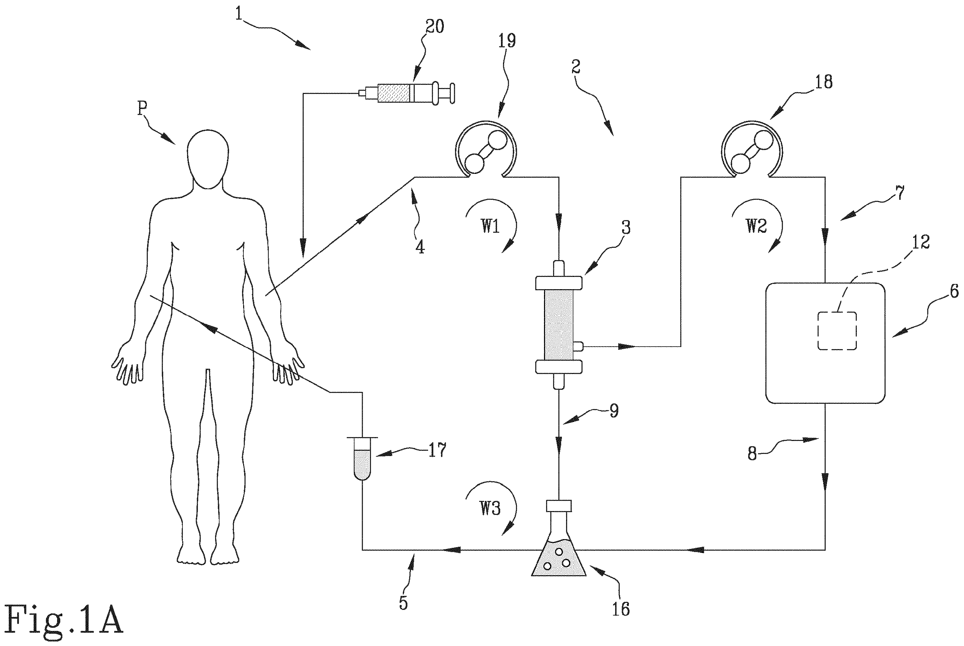

Figures 1A-1C illustrate a first embodiment of the invention.

The numeral 1' in Figure 1A denotes an apparatus for treating blood, according to this invention, which comprises an extracorporeal circuit 2. The extracorporeal circuit 2 comprises a filter 3 designed to separate the blood of a patient P into a plasma component and into a corpuscular component.

Preferably, the filter 3 consists of capillary tubes inside of which the blood flows.

According to an example, the filter 3 is responsible for the extraction by ultrafiltration of the plasma component.

The term "plasma component" means plasma water or whole plasma depending on the permeability of the capillaries used.

The extracorporeal circuit 2 comprises an arterial line 4, located upstream of the filter 3, in a direction W1 of feeding the blood of the patient P, for conveying the blood from the patient P to the filter 3.

The apparatus comprises, according to an embodiment, a pump 19 positioned along the arterial line 4 for moving the blood in the extracorporeal circuit 2.

According to an embodiment, the pump 19 is a hematic pump.

The extracorporeal circuit 2 may comprise a heparin pump 20 located at the arterial line 4 and upstream of the filter 3 in the direction of feeding blood to introduce heparin in the arterial line 4.

Advantageously, the heparin pump 20 makes it possible to avoid coagulations along the entire extracorporeal circuit 2.

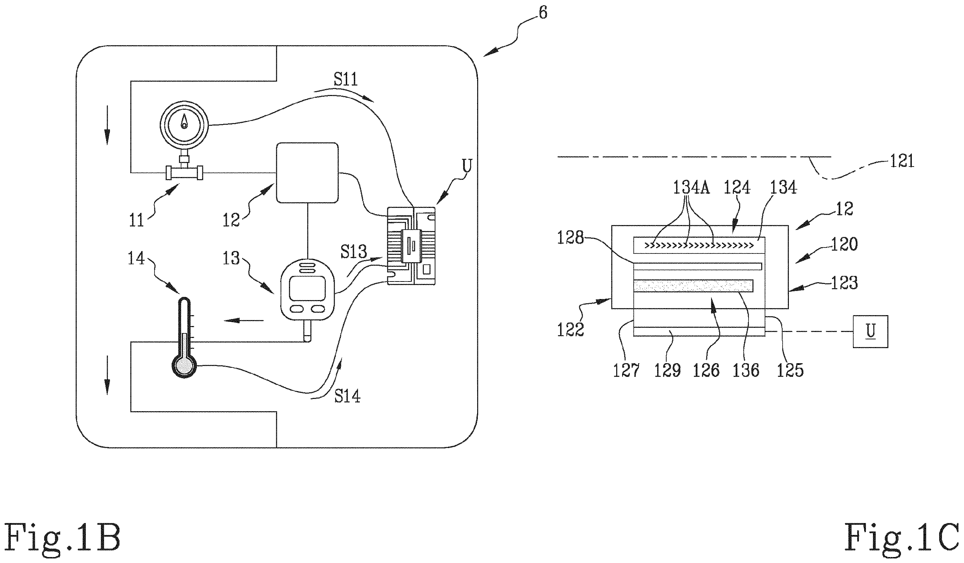

The extracorporeal circuit 2 comprises a treatment device 6 (illustrated in Figure 1B) located downstream of the filter 3, in a direction W2 of feeding the plasma component.

Inside the device 6 the plasma component is treated with a gas comprising ozone and/or oxygen to obtain a treated plasma component.

The extracorporeal circuit 2 comprises an infeed line 7 located between the filter 3 and the treatment device 6 for conveying the plasma component from the filter 3 to the treatment device 6.

The extracorporeal circuit 2 comprises an outfeed line 8 downstream of the treatment device 6, in the direction W2 of feeding the plasma component, for transferring the treated plasma component from the treatment device 6.

The treatment device 6 comprises an ozone generator 12 and is passed through by the plasma component.

The treatment device 6 is configured to generate in line in the extracorporeal circuit 2, in said plasma component ozone and/or oxygen. The oxygen is formed due to the strong instability of the ozone.

Advantageously, the device 6 allows an efficient dissolving of ozone and/or oxygen in the plasma component and guarantees the treatment of a large volume of blood in a single treatment session.

According to a preferred embodiment, the ozone generator 12 is an electrolysis ozone generator.

According to an embodiment, the ozone generator 12 comprises at least one electrolytic cell comprising one or more electrodes.

According to an example, the voltage of the electrolytic process for generating ozone is within the range of 1.5 V - 7 V.

According to an example, the intensity of electric current with which the electrolysis occurs in the generator 12 is within the range of 0.5 A - 3.0 A. The ozone generator 12, as illustrated in Figure 1C, comprises a main body 120 having a main direction of extension 121, an inlet 122 for the plasma component and an outlet 123 for the treated plasma component. The generator 12 comprises at least one cathode 124 inserted inside the main body 120 and having a respective negative power supply terminal 125.

The ozone generator 12 comprises at least one anode 126 inserted inside the main body 120 and having a respective positive power supply terminal 127.

The ozone generator 12 comprises an ion exchange membrane 128 positioned between the cathode 124 and the anode 126.

The membrane 128 extends mainly along said main direction of extension 121.

The ozone generator 12 comprises a power supply unit 129 for said cathode and anode.

The cathode 124, the anode 126 and the exchange membrane 128 intercept the plasma component which passes through the body 120.
the treatment device 6 comprises a computerised command and control unit U in communication with at least the power supply unit 129 for regulating the power supply voltage.

The unit U may comprise electronic hardware and/or software modules, located in a same control device or of the distributed type.

According to a preferred embodiment the cathode comprises a plate 134 which extends along the direction 121.

The plate 134 has a plurality of through holes 134A extending through a thickness of the plate 134.

Preferably, each through hole 134A is V-shaped along the main direction of extension 121

According to an embodiment, the anode 126 comprises a plate 136 which extends along the main direction of extension 121.

According to an embodiment, the treatment device 6 comprises a flow meter 11 positioned upstream of the ozone generator 12 in the feed direction W2 of the plasma component.

The flow meter 11 is in communication with the unit U and makes it possible to measure a flow of plasma component entering the treatment device 6.

The flow meter 11 generates a signal S11 signifying the flow measured. The unit U acts on the power supply unit 129 as a function of the signal S11.

Advantageously, controlling the supply voltage influences the quantity of ozone produced inside the plasma component.

Preferably, the flow (or flow rate) of the plasma component to be treated in the infeed line 7 is within the range of 50 ml/min - 400 ml/min.

According to an embodiment, the treatment device 6 comprises an ozone flow meter 13 which measures the concentration of ozone in the treated plasma component.

The flow meter 13 is positioned downstream of the ozone generator 12 in the feed direction W2 of the plasma component and is in communication with the unit U.

The flow meter 13 is configured to generate a signal S13 signifying the measured ozone concentration.

The unit U is configured to control the power supply as a function of the signal S13.

According to an embodiment, the treatment device 6 comprises a thermometer 14 configured for measuring a temperature of the treated plasma component.

The thermometer 14 is configured for generating a signal S14 signifying the measured temperature.

The thermometer 14 is preferably in communication with said unit U which is configured to control said power supply unit as a function of the signal S14.

According to an embodiment, the extracorporeal circuit 2 comprises a pump 18 positioned in the infeed line 7.

The pump 18 is configured for moving the plasma component towards the treatment device 6.

The pump 18 is in communication with the control unit U of the treatment device 6.

The extracorporeal circuit 2 comprises a mixing device 16 located downstream of the filter 3 in a direction W2 of feeding the corpuscular component, and of the treatment device 6 in the direction of feeding the plasma component.

The outfeed line 8 allows the treated plasma component to be transferred from the treatment device 6 to the mixing device 16.

The extracorporeal circuit 2 comprises a line 9 for transit of the corpuscular component from the filter 3 to the mixing device 16.

In other words, the mixing device 16 is in communication with the treatment device 6 by means of the outfeed line 8 and with the filter 3 by means of the line 9 for receiving, respectively, the treated plasma component and the corpuscular component.

The mixing device 16 being configured to mix the corpuscular component and the treated plasma component and to provide a treated blood at the outlet.

According to an embodiment, the mixing device 16 may be a device comprising a containment chamber in which the mixing occurs.

For example, the mixing device 16 may be any known connecting element (for example, with a "Y" shape) wherein there is at the inlet the corpuscular component and the treated plasma component and at the outlet the treated blood.

The extracorporeal circuit 2 comprises a venous line 5 located downstream of the mixing device 16, in a direction W3 of feeding the treated blood, for conveying and re-infusing the treated blood to the patient P.

The extra corporeal circuit 2 may comprise a device, not illustrated, for adjusting the temperature.

The temperature adjustment device is positioned preferably downstream of the treatment device 6, in the feed direction W2 of the plasma component, and it is in communication with the unit U.

According to an embodiment, the extracorporeal circuit 2 comprises a trap device 17 located downstream of the mixing device 16, in a direction W3 of feeding the treated blood.

The trap device 16 detects and traps bubbles of gas present inside the treated blood.

In this way, any air bubbles present inside the treated blood, which would result in serious problems for the health of the patient P if the treated blood with said air bubbles present in it were reinfused in the patient P, are promptly detected and suitably trapped or broken.

Advantageously, the apparatus 1 makes it possible to obtain a high level of solubilisation of ozone and/or gaseous oxygen in the plasma component by generating in line of said gas comprising ozone and/or oxygen, that is to say, the generation of said gas at said plasma component to be treated and inside the treatment device 6.

Advantageously, the apparatus 1 also makes it possible to treat a large volume of blood in a single treatment session, thanks to the configuration of the extracorporeal circuit 2 as described.

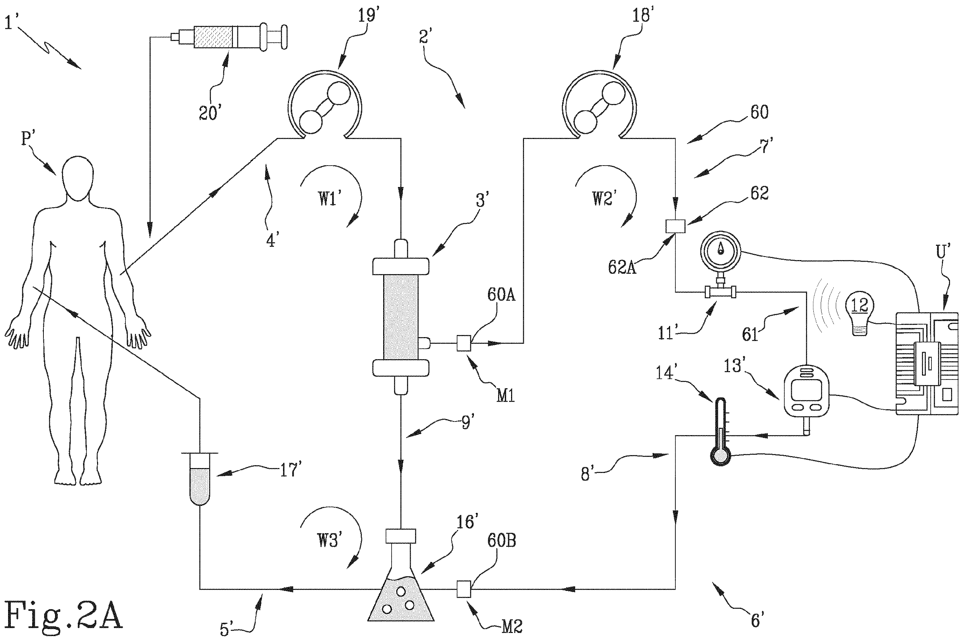

Figures 2A-2C illustrate a second embodiment of the invention.

The numeral 1' in Figure 2A denotes an apparatus for treating blood, according to this invention, which comprises an extracorporeal circuit 2'. The extracorporeal circuit 2' comprises a filter 3' designed to separate the blood of a patient P' into a plasma component and into a corpuscular component.

Preferably, the filter 3' consists of capillary tubes inside of which the blood flows.

According to an example, the filter 3' is responsible for the extraction by ultrafiltration of the plasma component.

The term "plasma component" means plasma water or whole plasma depending on the permeability of the capillaries used.

The extracorporeal circuit 2' comprises an arterial line 4, located upstream of the filter 3', in a direction W1' of feeding the blood of the patient P', for conveying the blood from the patient P' to the filter 3'.

The apparatus comprises, according to an embodiment, a pump 19' positioned along the arterial line 4' for moving the blood in the extracorporeal circuit 2'.

According to an embodiment, the pump 19' is a hematic pump.

The extracorporeal circuit 2' may comprise a heparin pump 20' located at the arterial line 4' and upstream of the filter 3' in the direction of feeding blood to introduce heparin in the arterial line 4'.

Advantageously, the heparin pump 20' makes it possible to avoid coagulations along the entire extracorporeal circuit 2'.

The extracorporeal circuit 2' comprises a treatment unit 6 located downstream of the filter 3', in a direction W2 of feeding the plasma component.

The treatment unit 6' comprises a treatment duct 60 having an inlet section 60A and an outlet section 60B.

The treatment duct 60 comprises an ozonizing stretch 61 wherein said plasma component is treated to obtain a treated plasma component.

The stretch 61 is a piece of pipe made of a material compatible with UV radiation.

According to an embodiment, the ozonizing section 61 is made of glass. According to an embodiment, the ozonizing section 61 is made of plastic material.

The plastic material from which the ozonizing section 61 is made must be compatible with UV radiation, preferably with UV-C radiation.

According to an embodiment, the ozonizing section 61 is made of PVC. The treatment unit 6' comprises first and second coupling means M1, M2 at the inlet section 60A and the outlet section 60B, respectively.

The treatment duct 60 is coupled to the filter 3' by the first coupling means M1.

The treatment duct 60 comprises preferably an inlet line 7' in communication with the filter 3' and located upstream of the ozonizing stretch 61, according to a feed direction W2' of the plasma component, to convey the plasma component from the filter 3' to the ozonizing stretch 61. The treatment duct 60 comprises preferably an outlet line 8' located downstream of the ozonising stretch 61, according to a feed direction W2' of the plasma component, to move away the plasma component treated from the ozonizing section 61.

The extracorporeal circuit 2' comprises a UV ray emitter 12' associated with the treatment unit 6'.

The emitter 12' is positioned in such a way as to emit a radiation in the direction of the ozonizing stretch 61 and to generate in line in the ozonizing stretch 61, ozone and/or oxygen in the plasma component in transit along the treatment duct 60.

According to a preferred embodiment, the UV ray emitter 12' is a UV-C ray emitter.

According to a preferred embodiment, the UV ray emitter 12' comprises a quartz lamp.

According to a preferred embodiment, the UV ray emitter 12' comprises an LED lamp.

The extracorporeal circuit 2' comprises a computerised command and control unit U' in communication with said UV ray emitter 12 to adjust an intensity of the radiation emitted.

According to an embodiment, the treatment unit 6 comprises a flow meter 11' positioned upstream of the emitter 12' in the feed direction W2' of the plasma component.

Preferably, the flow meter 11' is positioned along the treatment duct 60 upstream of the emitter 12' according to the feed direction W2' of the plasma component.

The flow meter 11' is in communication with the unit U' and allows a flow of the plasma component which flows in the treatment duct 60 to be measured.

The flow meter 11' generates a signal S11' signifying the flow measured. The unit U' receives and processes the signal S11'.

Preferably, the flow (or flow rate) of the plasma component passing in the ozonizing stretch 61 of the treatment duct 60 is within the range of 50 ml/min - 400 ml/min.

According to an embodiment, the treatment unit 6' comprises an ozone flow meter 13' which measures the concentration of ozone in the treated plasma component.

The flow meter 13' is positioned downstream of the emitter 12' in the feed direction W2' of the plasma component and is in communication with the unit U'.

The flow meter 13' is configured to generate a signal S13' signifying the measured ozone concentration.

The unit U' is configured to adjust the intensity of UV rays of the radiation emitted by the emitter 12' as a function of the second signal S13'. According to an embodiment, the treatment unit 6' comprises a thermometer 14' configured for measuring a temperature of the treated plasma component.

The thermometer 14' is configured for generating a signal S14' signifying the measured temperature.

The thermometer 14' is preferably in communication with the unit U' which receives and processes the signal S14'.

According to an embodiment, the treatment unit 6' comprises a pump 18'. According to an embodiment, the pump 18' is positioned in the inlet line 7'. The pump 18' is configured for moving the plasma component towards the ozonizing section 62.

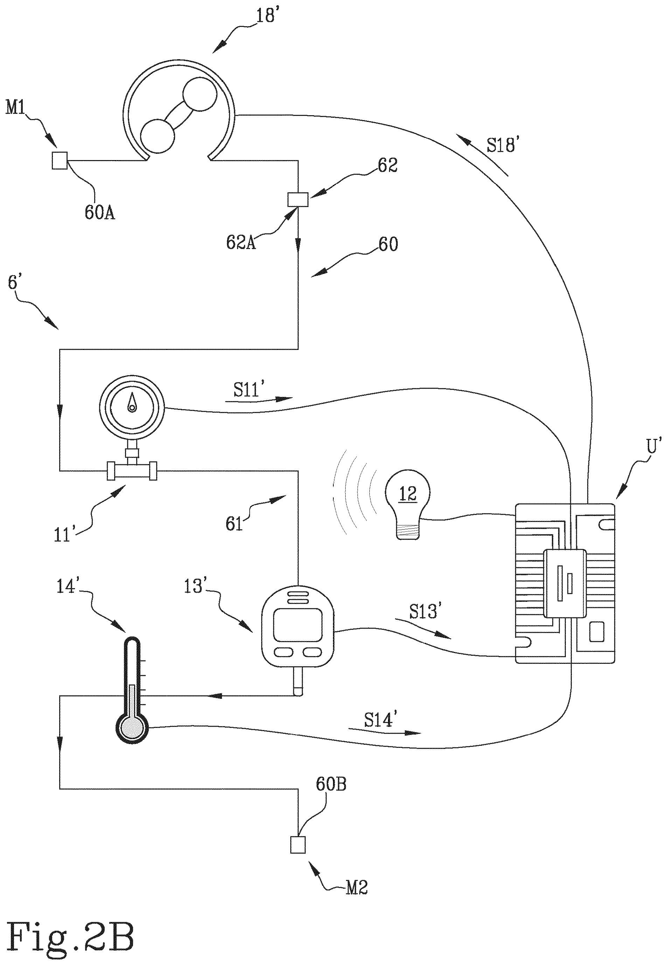

The pump 18' is in communication with the unit U' which controls it, using a signal S18', as a function of the signal S11' of the flow meter 11'. According to an embodiment, as illustrated in Figure 2B, the treatment duct comprises:
- a flow meter 11', positioned upstream of the UV ray emitter 12' according to the feed direction W2' of the plasma component, in communication with said computerised command and control unit U', configured for measuring a flow of plasma component in the treatment duct 60 and generating a first signal S11' representing the measured flow;
- a device 13' for measuring ozone in the treated plasma component, positioned downstream of the emitter 12' of UV rays according to the feed direction W2' of the plasma component, in communication with the computerised command and control unit U', the ozone measuring device 13' is configured for measuring a concentration of ozone in the treated plasma component and generating a second signal S13' representing the measured ozone concentration, the computerised command and control unit U' is configured for adjusting the intensity of UV rays of the radiation emitted as a function of the second signal S13';

- a thermometer 14' configured to measure a temperature of the treated plasma component and in communication with the computerised command and control unit U', the thermometer 14' is configured to generate a third signal S14' representing the measured temperature;
- a first pump 18' configured to move the plasma component in the treatment duct and in communication with the command and control unit U', the computerised command and control unit U' is configured to control the first pump 18' as a function of the first signal S11'.

According to a preferred embodiment, the treatment unit can be separated from the extracorporeal circuit.

The treatment unit 6' can therefore be replaced in the extracorporeal circuit 2'.

The extracorporeal circuit 2' comprises a mixing device 16' located downstream of the filter 3' in a direction W2' of feeding the corpuscular component, and of the treatment unit 6' in the direction of feeding the plasma component.

The mixing device 16' is configured for mixing the corpuscular component (incoming from the filter 3') and the treated plasma component (outgoing from the treatment unit) and providing a treated blood as output.

The treatment duct is coupled to the mixing device 16' by the second coupling means.

According to an embodiment, the mixing device 16' may be a device comprising a containment chamber in which the mixing occurs. For example, the mixing device 16' may be any known connecting element (for example, with a "Y" shape) wherein there is at the inlet the corpuscular component and the treated plasma component and at the outlet the treated blood.

The extracorporeal circuit 2' comprises a line 9' for transit of the corpuscular component from the filter 3' to the mixing device 16'.

The outfeed line 8' of the treatment unit 6' allows the treated plasma component to be transferred from the ozonizing section 61 to the mixing device 16'.

In other words, the mixing device 16' is in communication with the treatment unit 6' by means of the outfeed line 8' and with the filter 3' by means of the line 9' for receiving, respectively, the treated plasma component and the corpuscular component.

The extracorporeal circuit 2' comprises a venous line 5' located downstream of the mixing device 16', in a direction W3 of feeding the treated blood, for conveying and re-infusing the treated blood to the patient P'.

The extra corporeal circuit 2' may comprise a device, not illustrated, for adjusting the temperature.

The temperature adjustment device is positioned preferably downstream of the treatment unit 6, in the feed direction W2' of the plasma component, and it is in communication with the unit U'.

According to an embodiment, the extracorporeal circuit 2' comprises a trap device 17' located downstream of the mixing device 16', in a direction W3 of feeding the treated blood.

The trap device 17' detects and traps bubbles of gas present inside the treated blood.

In this way, any air bubbles present inside the treated blood, which would result in serious problems for the health of the patient P' if the treated blood with said air bubbles present in it were reinfused in the patient P', are promptly detected and suitably trapped or broken.

Advantageously, the apparatus 1' also makes it possible to treat a large volume of blood in a single treatment session, thanks to the configuration of the extracorporeal circuit 2' as described.

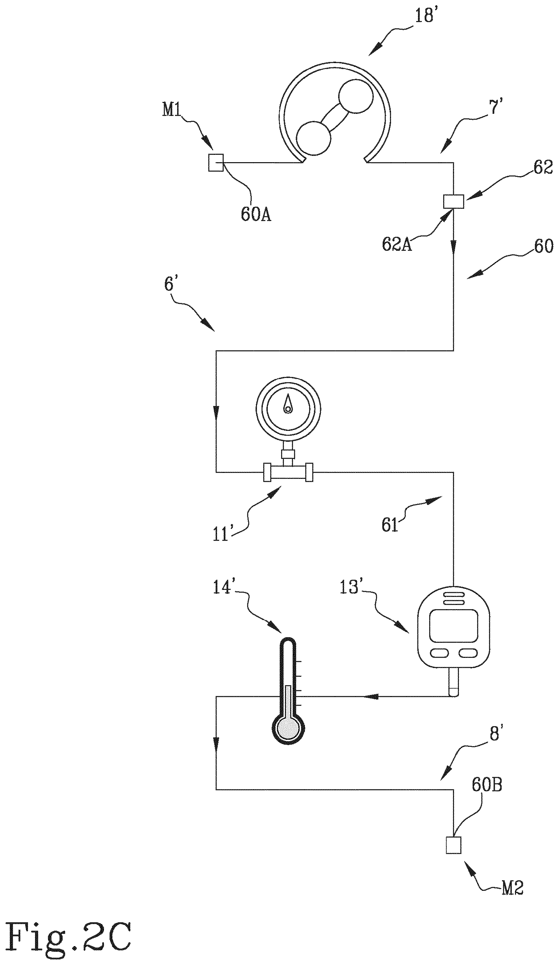

With reference to the second embodiment of the apparatus 1', the invention relates to a single-use treatment unit 6', illustrated in figure 2C, for a blood treatment apparatus according to the above-mentioned characteristics.

The single-use treatment unit 6' comprises the treatment duct 60 having the inlet section 60A and the outlet section 60B and comprising the ozonizing stretch 61 wherein the plasma component is treated to obtain a treated plasma component.

The ozonizing stretch 61 is made of a material compatible with UV radiation, in particular with UV-C radiation.

According to an embodiment, the ozonizing stretch 61 is made of glass or a plastic material such as PVC.

The single-use treatment unit 6 comprises first and second coupling means M1, M2 at the inlet section 60A and the outlet section 60B, respectively.

The treatment duct 60 can be coupled to the filter 3' and to the mixing device 16' of the blood treatment apparatus 1' using the first and the second coupling means M1, M2.

According to an embodiment, the single-use treatment unit 6' comprises:
- a flow meter 11' configured for measuring a flow of plasma component in the treatment duct 60 and generating a first signal S11' representing the flow measured;
- and/or a ozone measuring device 13' configured for measuring a concentration of ozone in the treated plasma component and generating a second signal S13' representing the measured ozone concentration.
- and/or a thermometer 14' configured to measure a temperature of the treated plasma component and generate a third signal S14' representing the measured temperature,
- and/or a pump 18' configured to move the plasma component in the treatment duct 60.

## Claims

1. A blood treatment apparatus (1), comprising
an extracorporeal circuit (2) comprising:
- a filter (3) designed to separate the blood of a patient (P) into a plasma component and into a corpuscular component,
- an arterial line (4), located upstream of the filter (3), in a direction (W1) of feeding the blood of the patient (P), for conveying the blood from the patient (P) to the filter (3),
- a treatment device (6) located downstream of the filter (3), in a direction (W2) of feeding the plasma component, and wherein said plasma component is treated with a gas comprising ozone and/or oxygen to obtain a treated plasma component,
- an infeed line (7) located between the filter (3) and the treatment device (6) for conveying the plasma component from the filter (3) to the treatment device (6),
- a mixing device (16) located downstream of the filter (3) in a direction of feeding the corpuscular component, and of the treatment device (6) in the direction of feeding the plasma component,
- an outfeed line (8) downstream of the treatment device (6), in the direction (W2) of feeding the plasma component, for transferring the plasma component treated by the treatment device (6) to the mixing device (16),
said mixing device (16) being in communication with the treatment device (6) by means of the outfeed line (8) and with the filter (3) for receiving the treated plasma component and the corpuscular component,
said mixing device (16) being configured to mix the corpuscular component and the treated plasma component and provide at the outlet a treated blood,
the extracorporeal circuit (2) comprising
a venous line (5) located downstream of the mixing device (16), in a direction (W3) of feeding the treated blood, for conveying the treated blood to the patient (P),
the treatment device (6) comprising an ozone generator (12) passed through by said plasma component, configured for generating in line in the extracorporeal circuit (2), ozone and/or oxygen in said plasma component.

2. The apparatus according to claim 1, wherein the ozone generator (12) is an electrolysis ozone generator.

3. The apparatus according to claim 1 or 2, wherein said ozone generator (12) comprises
- a main body (120) having a main direction of extension (121), an inlet (122) for the plasma component and an outlet (123) for the treated plasma component,
- at least one cathode (124) inserted inside the main body and having a respective negative power supply terminal (125)
- at least one anode (126) inserted in the main body and having a respective positive power supply terminal (127)
- an ion exchange membrane (128) positioned between the cathode (124) and the anode (126)
- a power supply unit (129) for said cathode (124) and anode (126),
- said cathode (124), anode (126) and exchange membrane (128) intercepting the plasma component passing through the main body (120), said treatment device (6) comprising a computerised command and control unit (U) in communication with the power supply unit (129) to adjust a supply voltage.

4. The apparatus according to claim 3, wherein said cathode comprises a first plate (134) extending along said main direction of extension (121) and having a plurality of through holes (134A) through a thickness of the first plate (134)
and wherein said anode (126) comprises a second plate (136) extending along said main direction of extension (121), said membrane (128) extending mainly along said main direction of extension (121) and being positioned between said first and second plates (134, 136).

5. The apparatus according to claim 4, wherein each through hole (134A) of the plurality of through holes (134A) is V-shaped.

6. The apparatus according to any one of claims 3 to 5, wherein said treatment device (6) comprises
- a flow meter (11) positioned upstream of the ozone generator (12) in the direction (W2) of feeding the plasma component and in communication with said computerised command and control unit (U), said flow meter (11) being configured to measure a flow of plasma component entering the treatment device (6) and generate a first signal (S11) signifying the flow measured, said computerised command and control unit (U) being configured to control said power supply unit (129) as a function of said first signal (S11),
- and/or
a flow meter (13) for measuring ozone in said treated plasma component positioned downstream of the ozone generator (12) in the direction (W2) of feeding the plasma component and in communication with said computerised command and control unit (U), said ozone flow meter (13) being configured to measure a concentration of ozone in the treated plasma component and generate a second signal (S13) signifying the ozone concentration measured, said computerised command and control unit (U) being configured to control said power supply unit (129) as a function of said second signal (S13),
- and/or
a thermometer (14) configured to measure a temperature of the treated plasma component and in communication with said computerised command and control unit (U), said thermometer (14) being configured to generate a third signal (S14) signifying the temperature measured, said computerised command and control unit (U) being configured to control said power supply unit (129) as a function of said third signal (S14).

7. The apparatus according to any one of claims from 3 to 6, wherein said extracorporeal circuit (2) comprises:
- a first pump (18) positioned in the infeed line (7), said first pump (18) being configured to move the plasma component towards the treatment device (6) and being in communication with the control unit (U) of the treatment device (6).
- and/or a second pump (19) positioned in the arterial line (4), said second pump (19) being configured to move the blood towards the filter (3) and being in communication with the command and control unit (U) of the treatment unit (6).
- and/or a device for adjusting the temperature positioned downstream of the treatment unit (6), in the feed direction (W2) of the plasma component, said device for adjusting the temperature being in communication with the command and control unit (U) of the treatment unit (6).
- and/or a trap device (17) positioned downstream of the mixing device (16), in a direction (W3) of feeding treated blood, said trap device (16) being configured for detecting and trapping gas bubbles present inside the treated blood.

8. An apparatus for blood treatment comprising an extracorporeal circuit (2') comprising:
- a filter (3') designed to separate the blood of a patient (P') into a plasma component and into a corpuscular component,
- an arterial line (4'), located upstream of the filter (3'), in a direction (W1') of feeding the blood of the patient (P'), for conveying the blood from the patient (P') to the filter (3'),
- a treatment unit located downstream of the filter (3'), in a feed direction (W2') of the plasma component, said treatment unit (6') comprising a treatment duct (60) having an inlet section (60A) and an outlet section (60B), said treatment duct (60) comprising an ozonizing stretch (61) wherein said plasma component is treated to obtain a treated plasma component, said treatment unit (60) comprising first and second coupling means (M1, M2) at, respectively, the inlet section (60A) and the outlet section (60B), the treatment duct (60) being coupled to the filter (3') by means of the first coupling means (M1),
- a mixing device (16') in communication with the outlet section of the treatment duct and with the filter (3') said mixing device (16') being configured for mixing the corpuscular component and the treated plasma component and providing at the outlet a treated blood, the treatment duct being coupled to the mixing device (16') by means of the second coupling means (M2),
the extracorporeal circuit (2') comprising
- a venous line (5') located downstream of the mixing device (16'), in a direction (W3') of feeding the treated blood, for conveying the treated blood to the patient (P'),
- a UV ray emitter (12') associated with the treatment unit (6') in such a way as to emit a radiation in the direction of the ozonizing stretch (61) and to generate in line in said ozonizing stretch (61), ozone and/or oxygen in said plasma component in transit along the treatment duct (60),
- a computerised command and control unit (U') in communication with said UV ray emitter (12') to adjust an intensity of the radiation emitted.

9. The apparatus according to claim 8, wherein the UV ray emitter (12') is a UV-C ray emitter (12') and/or said ozonising stretch (61) is made of glass or of plastic material.

10. The apparatus according to claim 8 or 9, wherein said UV ray emitter (12') comprises a quartz lamp or an LED lamp.

11. The apparatus according to any of the claims from 8 to 10, wherein said treatment unit (6') comprises
- a flow meter (11'), positioned upstream of the UV ray emitter (12') according to the feed direction (W2') of the plasma component, in communication with said computerised command and control unit (U'), said flow meter (11') being configured for measuring a flow of plasma component in the treatment duct (6') and generating a first signal (S11') representing the measured flow
- and/or a device (13') for measuring ozone in said treated plasma component, positioned downstream of the emitter (12') of UV rays (12') according to the feed direction (W2') of the plasma component, in communication with said computerised command and control unit (U'), said ozone measuring device (13') being configured for measuring a concentration of ozone in the treated plasma component and generating a second signal (S13') representing the measured ozone concentration, said computerised command and control unit (U') being configured for adjusting the intensity of UV rays of the radiation emitted as a function of said second signal (S13'),
- and/or a thermometer (14') configured to measure a temperature of the treated plasma component, said thermometer (14') being in communication with said computerised command and control unit (U') and being configured to generate a third signal (S14') representing the measured temperature.

12. The apparatus according to any one of claims 8 to 10 and according to claim 11, wherein said treatment unit (6') comprises
- a first pump (18'), said first pump (18') being configured to move the plasma component in the treatment duct (60') and being in communication with the command and control unit (U),
- the computerised command and control unit (U') being configured to control said first pump (18') as a function of said first signal (S11').
- and/or a scrubber (62) for oxygen positioned between the inlet section (60A) and the ozonizing stretch (61), said scrubber (62) being preferably positioned downstream of the first pump (18) in a feed direction (W2) of the plasma component.

13. The apparatus according to any one of claims 8 to 12, wherein said extracorporeal circuit (2') comprises:
- a second pump (19') positioned in the arterial line (4'), said second pump (19') being configured to move the blood towards the filter (3') and being in communication with the command and control unit (U),
- and/or a device for adjusting the temperature positioned downstream of said treatment unit (6'), in the feed direction (W2') of the plasma component, said device for adjusting the temperature being in communication with the command and control unit (U'),
- and/or a trap device (17') positioned downstream of the mixing device (16'), in a direction (W3') of feeding treated blood, said trap device (16') being configured for detecting and trapping gas bubbles present inside the treated blood.

14. The apparatus according to any one of claims 8 to 13, wherein said treatment duct (60) comprises:
- a flow meter (11'), positioned upstream of the UV ray emitter (12') according to the feed direction (W2') of the plasma component, in communication with said computerised command and control unit (U'), said flow meter (11') being configured for measuring a flow of plasma component in the treatment duct (60) and generating a first signal (S11') representing the measured flow,
- a device (13') for measuring ozone in said treated plasma component, positioned downstream of the emitter (12') of UV rays according to the feed direction (W2') of the plasma component, in communication with said computerised command and control unit (U'), said ozone measuring device (13') being configured for measuring a concentration of ozone in the treated plasma component and generating a second signal (S13') representing the measured ozone concentration, said computerised command and control unit (U') being configured for adjusting the intensity of UV rays of the radiation emitted as a function of said second signal (S13'),
- a thermometer (14') configured to measure a temperature of the treated plasma component, said thermometer (14') being in communication with said computerised command and control unit (U') and being configured to generate a third signal (S14') representing the measured temperature,
- a first pump (18') configured to move the plasma component in the treatment duct (60), said first pump (18') being in communication with the command and control unit (U'),
the computerised command and control unit (U') being configured to control said first pump (18') as a function of said first signal (S11')
- a scrubber (62) for oxygen positioned between the inlet section (60A) and the ozonizing stretch (61), said scrubber (62) being preferably positioned downstream of the first pump (18') in a feed direction (W2') of the plasma component.

15. The apparatus according to claim 14, wherein said treatment unit (6') can be separated from the extracorporeal circuit (2'), said treatment unit (6') being replaceable in the extracorporeal circuit (2').

16. A single-use treatment unit for a blood treatment apparatus according to any one of claims 8 to 15, comprising:
- the treatment duct (60) having the inlet section (60A) and the outlet section (60B) and comprising the ozonizing stretch (61) wherein said plasma component is treated to obtain a treated plasma component, said ozonizing stretch (61) being made of a material compatible with UV radiation, in particular with UV-C radiation.
- first and second coupling means (M1, M2), respectively at the inlet section (60A) and the outlet section (60B), the treatment duct being connectable to the filter (3') and to the mixing device (16') of the blood treatment apparatus using the first and the second coupling means (M1, M2).

17. The unit according to claim 16, wherein the ozonising stretch (61) is made of glass or plastic material.

18. The unit according to claim 16 or 17, wherein said treatment duct (60) comprises:
- a flow meter (11') configured for measuring a flow of plasma component in said treatment duct (60) and generating a first signal (S11') representing the flow measured;
- and/or ozone measuring device (13') configured for measuring a concentration of ozone in the treated plasma component and generating a second signal (S13') representing the measured ozone concentration;
- and/or a thermometer (14') configured to measure a temperature of the treated plasma component and generate a third signal (S14') representing the measured temperature,
- and/or a pump (18') configured to move the plasma component in the treatment duct (60);
- and/or a scrubber (62) for oxygen positioned between the inlet section (60A) and the ozonizing stretch (61), said scrubber (62) being preferably positioned downstream of the first pump (18') in a feed direction (W2') of the plasma component.
